# EUROPEAN PATENT SPECIFICATION

(11) **EP 1 990 140 B1**
(45) Date of publication and mention of the grant of the patent: **18.07.2012**
(21) Application number: 07714546.4
(22) Date of filing: 20.02.2007
(51) Int. Cl.: B26B 3/00, A61B 17/32, B26B 29/02

(54) **EDGED TOOL**
SCHARFES WERKZEUG
OUTIL À ARÊTE

(30) Priority: 27.02.2006 JP 2006050529; 06.03.2006 JP 2006059521
(43) Date of publication of application: 12.11.2008
(73) Proprietor: Feather Safety Razor Co., Ltd., Osaka-shi, Osaka 531-0075 (JP)
(72) Inventor: UENO, Shoji, Seki-shi, Gifu-ken 501-3881 (JP); TERAZAWA, Yoshihiro, Seki-shi, Gifu-ken 501-3881 (JP)
(74) Representative: Viering, Jentschura & Partner
(86) International application number: PCT/JP2007/053020
(87) International publication number: WO 2007/097298

(56) References cited:
- WO-A-93/24063
- JP-A- 2003 339 723
- JP-A- 2005 040 573
- JP-A- 2006 503 682
- JP-Y2- 61 002 570
- JP-Y2- 62 013 606
- US-A- 4 576 164
- US-A- 4 735 202
- US-A- 5 254 128
- US-A- 5 275 606
- US-A- 5 391 177
- US-A1- 2003 093 100

## Description

### TECHNICAL FIELD

The present invention relates to an edged tool.

### BACKGROUND ART

Conventionally, scalpels that have been disclosed in Patent Documents 1 to 4 are publicly known as an edged tool. These scalpels are all configured so that the blade body thereof is protected when not in use or the scalpels can be safely handed from an assistant to a physician during surgical procedures.

Specifically, in the scalpel disclosed in Patent Document 1, a sheath tube, the distal end portion of which is opened, is fitted into the outer periphery of a supporting rod having a cutting blade so as to slide freely. The sheath tube is configured in such a manner that the cutting blade can be selectively placed at two positions, that is, a position at which the cutting blade is covered by the sheath tube and a position at which the cutting blade is exposed.

In the scalpel disclosed in Patent Document 2, a fixing member having a scalpel portion is mounted at the distal end of a sliding rod. The sliding rod slides inside a distal-end cylindrical portion and a holder main body, by which the fixing member slides coaxially inside the distal-end cylindrical portion and the holder main body, and the scalpel portion is exposed from the distal-end cylindrical portion or accommodated into the distal end cylindrical portion.

In the scalpel disclosed in Patent Document 3, when a pressing member is pressed by a thumb, with the holder main body grasped, a first sliding rod and a second sliding rod slide, allowing the scalpel fixing member to project from the distal end cylindrical portion of the holder main body. This state is kept by actions of a cam mechanism internally mounted on the holder main body. Then, the thumb is used to press again the pressing member, by which the scalpel fixing member is accommodated into the distal-end cylindrical portion of the holder main body.

In the scalpel disclosed in Patent Document 4, a protective guard is placed at the distal end portion of a handle. The protective guard is slidable between two positions, that is, a position at which a blade mounted at the distal end of the handle is covered and a position at which the blade is exposed. Locking means, which is a member separate from the protective guard, is mounted between the handle and the protective guard. Then, the locking means is manipulated, by which the protective guard can be retained at a certain position or released at the position.

US 5 254 128 A discloses an edged tool with a protective cover, comprising a handle main body having a blade body at a distal end, wherein the protective cover is placed on an outer periphery of the handle main body, so as to slide freely along an axial direction of the handle main body between a position at which the protective cover covers the blade body and a position at which the blade body is exposed.

US 4 735 202 A discloses a microsurgical knife 10 having a guard 20 detachably attached therewith. When the guard 20 is attached with the knife 10, a projection 22 of the knife 10 is engaged with a groove 37 of the guard 20. However, this document also fails to disclose and teach the above-mentioned claimed feature.
Patent Document 1: Japanese Examined Utility Model Publication No. 61-2570
Patent Document 2: Japanese Examined Utility Model Publication No. 62-13606
Patent Document 3: Japanese Examined Utility Model Publication No. 62-13607
Patent Document 4: Japanese Laid-Open Patent Publication No. 6-133979

### DISCLOSURE OF THE INVENTION

However, on lines 25 to 27 in the left column on page 1 in Patent Document 1, such a description is made that "favorable results are often obtained in a case where the rear end portion of a sheath tube is substantially aligned with the rear end portion of a supporting rod at the exposure site of a cutting blade." With reference to this description, in Patent Document 1, when the cutting blade is exposed, the rear end portion is covered with the sheath tube from the central part of a supporting rod. Therefore, when the sheath tube moves from a position at which the cutting blade is covered to a position at which the cutting blade is exposed, it is necessary that the supporting rod is gripped with the right hand (or the left hand), and at the same time, the sheath tube is gripped with the left hand (or the right hand). Further, it is necessary to use both hands when the sheath rod moves from a position at which the cutting blade is exposed to a position at which the cutting blade is covered.

On lines 1 to 7 in the fourth column on page 2 in Patent Document 2, such a description is made that both hands are used to allow a holder main body and a sliding rod to slide in a relative manner, by which a scalpel portion is exposed from the holder main body. In other words, in Patent Document 2 as well, it is necessary to use both hands in exposing a scalpel portion or covering the scalpel portion by the holder main body.

According to Patent Document 3, it is possible to expose the blade body of a scalpel and accommodate the blade body into a holder main body by using one hand. However, it is necessary to mount a spring member between the holder main body and a second sliding rod and also provide a cam mechanism, thereby making the configuration complicated or increasing the number of parts and the steps for assembly to result in an increased cost.

According to Patent Document 4, a protective guard can be manipulated by one hand, but it is necessary to provide locking means as a separate member for retaining the protective guard at a selected position. Therefore, the number of parts and the steps for assembly are increased to result in an increased cost.

An objective of the present invention is to provide an edged tool with a protective cover that requires no separate member for locking the protective cover on a handle main body, allows a protective cover protecting a blade body to be easily manipulated by one hand, and also retains the blade body both in an exposed state and in a covered state.

According to the present invention, an edged tool as claimed in claim 1 is provided. The dependent claims show some examples of such an edged tool.

In one aspect of the present invention, there is provided an edged tool with a protective cover. The edged tool has a handle main body having the blade body at the distal end. The protective cover is placed on the outer periphery of the handle main body so as to slide freely between a position at which the protective cover covers the blade body and a position at which the blade body is exposed. The handle main body is provided with a grip portion formed so as to be held by one hand and a supporting portion integrally coupled to the grip portion to support the protective cover so as to allow the protective cover to slide freely on the outer periphery. A projection is formed on the outer periphery of the supporting portion. A pair of locking portions are formed on the protective cover. The projection makes an elastic engagement with the locking portions when the protective cover is selectively placed at the covering position and the exposing position.

According to the above configuration, since a member for retaining the protective cover is formed integrally with the protective cover at a position at which the blade body is

covered or at a position at which the blade body is exposed, the number of parts is decreased. For this reason, there is no necessity for providing a separate member for locking the protective cover on the handle main body. Further, it is possible to manipulate the protective cover for protecting the blade body easily by one hand and also retain the blade body both in a state where it is exposed and in a state where it is covered.

It is preferable that a slit extending along the axial direction of the protective cover is formed on the protective cover and the projection enters and is engaged with the slit so as to be movable relative to the slit. A pair of locking portions are formed on both ends of the slit. At least one of the locking portions is formed in such a manner so as to extend in a direction intersecting a direction in which the slit extends and provided with a notch which allows the projection to enter and be engaged with the notch. The protective cover is placed so as to be rotatable around the axis thereof.

According to the above configuration, since there is formed a notch which extends in a direction intersecting a direction in which the slit extends, a user of the edged tool allows the protective cover to rotate around the axis thereof, thereby causing the projection to enter and be engaged with the notch. In other words, through such a simple manipulation that the thumb of one hand holding the grip portion is placed on the protective cover, allowing the protective cover to rotate around the axis thereof, the projection can enter and be engaged with the notch of the locking portion.

It is preferable that the locking portion having the notch is formed only at the end portion close to the grip portion in the slit. According to this configuration, the locking portion having the notch extending in a direction intersecting a direction in which the slit extends is formed only at the end portion adjacent to the grip portion on the slit. In other words, when the protective cover is placed at a position at which the blade body is covered, the projection enters and is engaged with the notch of the locking portion at the end portion. In this state, even when a force directing to the grip portion is applied to the protective cover, there is no chance that the protective cover is removed from the notch of the locking portion, thus making it possible to reliably retain the protective cover at a position at which the blade body is covered.

On the other hand, when the protective cover is moved from a position at which the blade body is covered, a user performs a two-stage motion by using the thumb of one hand holding the grip portion that the protective cover is manipulated so as to rotate, thereby removing the projection from the notch, thereafter, allowing the protective cover to move to a direction of the grip portion. Therefore, only the two-stage motion by the thumb of one hand holding the grip portion is performed, by which the protective cover retained at a position at which the blade body is covered can be easily moved to a position at which the blade body is exposed.

It is preferable that a locking portion having the notch is formed at the end portion close to the grip portion and at the end portion spaced away from the grip portion in the slit. According to this configuration, the same advantages can be obtained as described above. Further, when the protective cover is placed at a position at which the blade body is covered, the projection enters and is engaged with the notch of the locking portion at the end portion spaced away from the grip portion. In this state, even when a force is applied in the opposite direction of the grip portion to the protective cover, there is no chance that the protective cover is removed from the notch of the locking portion, thus making it possible to reliably retain the protective cover at a position at which the blade body is exposed.

On the other hand, when the protective cover is moved from a position at which the blade body is exposed, a user performs a two-stage motion by using the thumb of one hand holding the grip portion that the protective cover is manipulated so as to rotate, thereby removing the projection from the notch, thereafter, allowing the protective cover to move to a direction opposite to the grip portion. Therefore, only the two-stage motion by the thumb of one hand holding the grip portion is performed, by which the protective cover retained at a position at which the blade body is covered can be easily moved to a position at which the blade body is exposed and also the protective cover retained at a position at which the blade body is exposed can be easily moved to a position at which the blade body is covered.

It is preferable that the locking portion is provided with a notch and an elastic engagement piece forming a peripheral part of the notch. According to this configuration, the elastic engagement piece makes an elastic engagement with a projection to retain the projection, by which the protective cover is retained at a position at which the blade body is covered or at a position at which the blade body is exposed.

It is preferable that a knurling or a manipulating projection is formed on the peripheral surface of the protective cover. According to this configuration, the thumb touches the knurling or the manipulating projection, with the grip portion held by one hand, thereby the protective cover can be easily manipulated. When the thumb is used to perform a two-stage motion, the thumb touches the knurling or the manipulating projection, by which the protective cover can be easily manipulated so as to rotate.

It is preferable that the supporting portion is smaller in cross section than the grip portion, and the proximal end portion of the protective cover is in contact with the end face of the grip portion when the protective cover is placed at the exposing position. According to this configuration, when the protective cover is placed at a position at which the blade body is exposed, the proximal end portion of the protective cover is in contact with the end face of the grip portion. As a result, since no clearance is developed between the grip portion and the protective cover on manipulation of an edged tool, no hindrance is found on the fingers gripping the handle main body and the protective cover on manipulation of the edged tool, thereby the edged tool can be manipulated easily. Assuming that the proximal end portion of the protective cover is not in contact with the end face of the grip portion to develop a clearance between them, the clearance will be a hindrance to the fingers for gripping the handle main body and the protective cover, thus making it difficult to manipulate the edged tool.

In another aspect of the present invention, a medical edged tool including a handle main body having a blade body at the distal end is provided. A protective cover is placed on an outer periphery of the handle main body, so as to be movable between a covering position at which the protective cover covers a blade body and an exposing position at which the blade body is exposed. A deflection restraining member is mounted on the inner wall surface of the protective cover so as to be in contact with the blade body when the protective cover is placed at the covering position or so as to be in contact with the blade body by being positioned within a trace of the blade body when the blade body deflects.

According to this configuration, when load is applied to the blade body, for example, when an edged tool is being transported or the edged tool is dropped, the deflection restraining member of the protective cover is in contact with the blade body or positioned within the trace of the blade body when the blade body deflects. Therefore, it is possible to restrain or prevent the deflecting of the blade body and also restrain the damage of the tip of cutting edge. Further, when the protective cover is moved to expose the blade body, there is nothing on the protective cover that blocks visual observation of the blade body, thus there is no chance that the visual observation of the blade body is obstructed by the protective cover when the edged tool is in use.

It is preferable that the blade body is a bent-type blade body at which the blade body is bent to have an inner surface and an outer surface due to the bending and the deflection restraining member is formed so as to be in contact with the inner surface of the blade body when the protective cover is placed at the covering position or so as to be in contact with the inner surface by being positioned within a trace of the blade body when the blade body deflects. According to this configuration, the bent-type blade body can be used to obtain the advantages similar to those described above.

It is preferable that an allowance portion for allowing the deflection restraining member to move when the protective cover is placed at the exposing position is formed on the outer periphery of the handle main body. According to this configuration, when the protective cover is moved to the exposing position, the protective cover does not interfere with the handle main body or the protective cover does not be affected by the movement when manipulated.

It is preferable that lock means for locking the movement of the protective cover so as not to be unlocked in a state where the blade body is covered by the protective cover is mounted on the outer periphery of the handle main body. According to this configuration, the protective cover locked by the lock means so as not to be unlocked cannot be moved, by which the blade body is kept covered to handle a medical edged tool safely.

It is preferable that when the protective cover is locked by the lock means, the deflection restraining member is placed at a position exceeding the distal end of the blade body. According to this configuration, when the protective cover is locked by the lock means, a deflection restraining member is positioned outside the blade body, thereby the deflection restraining member prevents foreign matter or the like from moving into the protective cover. In the present application, such a description that the deflection restraining member is placed at a position exceeding the distal end of the blade body means that the deflection restraining member is positioned outside the distal end of the blade body in a direction in which the blade body extends.

### BRIEF DESCRIPTION OF THE DRAWINGS

Fig. 1(a) is a front elevational view showing a surgical scalpel in a state where a blade body of a first embodiment is exposed;
Fig. 1(b) is a front elevational view showing the surgical scalpel in a state where the blade body is covered;
Fig. 2(a) is a side elevational view showing a handle main body;
Fig. 2(b) is a plan view showing a supporting portion of the handle main body;
Fig. 3(a) is a side elevational view showing a protective cover;
Fig. 3(b) is a front elevational view showing the protective cover;
Fig. 4(a) is a front elevational view showing major parts of a surgical scalpel of a second embodiment;
Fig. 4(b) is a front elevational view showing major parts of a surgical scalpel of a third embodiment;
Fig. 5(a) is a front elevational view showing a scalpel for ophthalmic surgery in a state where a blade body of a fourth embodiment is exposed;
Fig. 5(b) is a front elevational view showing the scalpel for ophthalmic surgery in a state where the blade body is covered;
Fig. 6(a) is a side elevational view showing a handle main body;
Fig. 6(b) is a plan view showing a supporting portion of the handle main body;
Fig. 7(a) is a side elevational view showing a protective cover;
Fig. 7(b) is a front elevational view showing the protective cover;
Fig. 8 is a cross-sectional view showing major parts of the scalpel for ophthalmic surgery in a state where the blade body is exposed;
Fig. 9 is a cross-sectional view showing major parts of the scalpel for ophthalmic surgery in a state where the blade body is covered;
Fig. 10 (a) is a front elevational view showing a scalpel for ophthalmic surgery in a state where a blade body of a fifth embodiment is covered;
Fig. 10(b) is a front elevational view showing the scalpel for ophthalmic surgery in a state where the blade body is exposed;
Fig. 10(c) is a front elevational view showing the scalpel for ophthalmic surgery in a state where the protective cover is placed at a lock position;
Fig. 11(a) is a front elevational view showing a supporting portion of the handle main body;
Fig. 11(b) is a plan view showing the supporting portion of the handle main body;
Fig. 11(c) is a cross-sectional view, with a part cut away, showing the supporting portion of the handle main body;
Fig. 12(a) is a plan view showing a protective cover;
Fig. 12(b) is a front elevational view showing the protective cover;
Fig. 12(c) is a front cross-sectional view showing the protective cover;
Fig. 12(d) is a bottom view showing the protective cover;
Fig. 12(e) is a side elevational view showing the protective cover.
Fig. 13(a) is a cross-sectional view taken along line 13a-13a in Fig. 10(a);
Fig. 13(b) is a cross-sectional view taken along line 13b-13b in Fig. 10(b);
Fig. 13(c) is a cross-sectional view taken along line 13c-13c in Fig. 10(c);
Fig. 14(a) is a cross-sectional view taken along line 14a-14a in Fig. 11(b);
Fig. 14(b) is a cross-sectional view taken along line 14b-14b in Fig. 11(b);
Fig. 14(c) is a cross-sectional view taken along line 14c-14c in Fig. 11(b);
Fig. 14(d) is a cross-sectional view taken along line 14d-14d in Fig. 11(b); and
Fig. 14(e) is a cross-sectional view taken along line 14e-14e in Fig. 11(b).

### BEST MODE FOR CARRYING OUT THE INVENTION

### (First Embodiment)

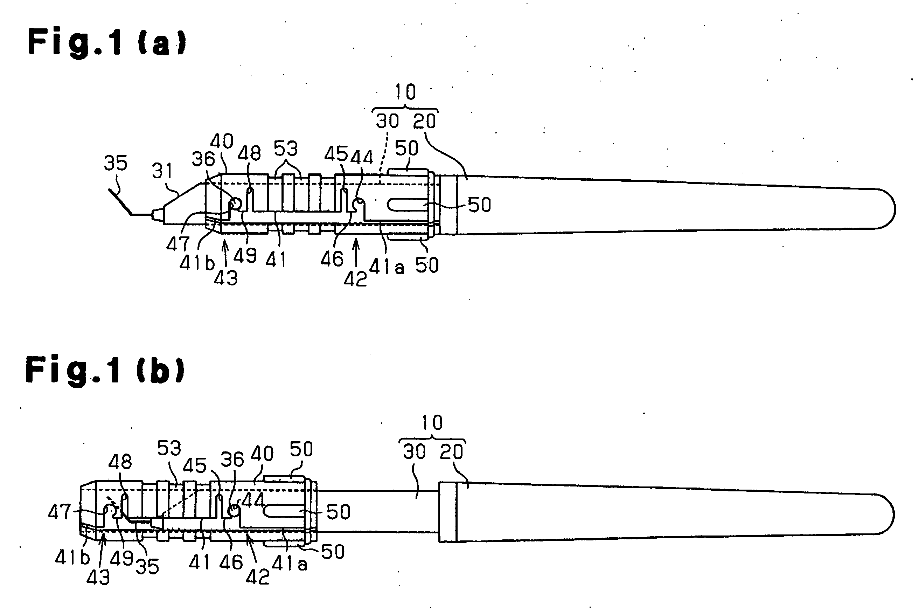

Hereinafter, an explanation will be made of an edged tool with a protective cover according to a first embodiment of the present invention, which is a surgical scalpel, by referring to Figs. 1 to 3. As shown in Figs. 1(a) and 1(b), a handle main body 10 of the surgical scalpel is made of a grip portion 20 and a supporting portion 30 mounted at the distal end of the grip portion 20 coaxially with the grip portion 20. The grip portion 20 has a length that can be gripped by one hand and a circular cross section. The grip portion 20 is set to be gradually smaller in outer diameter toward the proximal end of the grip portion 20 (the right end in Fig. 1(a)). There is no limitation on a material of the handle main body 10, and the handle main body 10 is made of, for example, a synthetic resin or a metal such as stainless steel.

The supporting portion 30 is formed in a cylindrical shape except for the distal end portion thereof and smaller in outer diameter than the distal end portion of the grip portion 20. In other words, the supporting portion 30 is smaller in cross section than the grip portion 20. The distal end portion of the grip portion 20 is the largest in outer diameter in a whole part of the grip portion 20. An inclined surface 31 which inclines from the proximal end portion to the distal end portion is formed on the peripheral surface at the distal end of the supporting portion 30. A blade body 35 is mounted at a site made eccentric from the axis of the supporting portion 30 on the distal end face of the supporting portion 30.

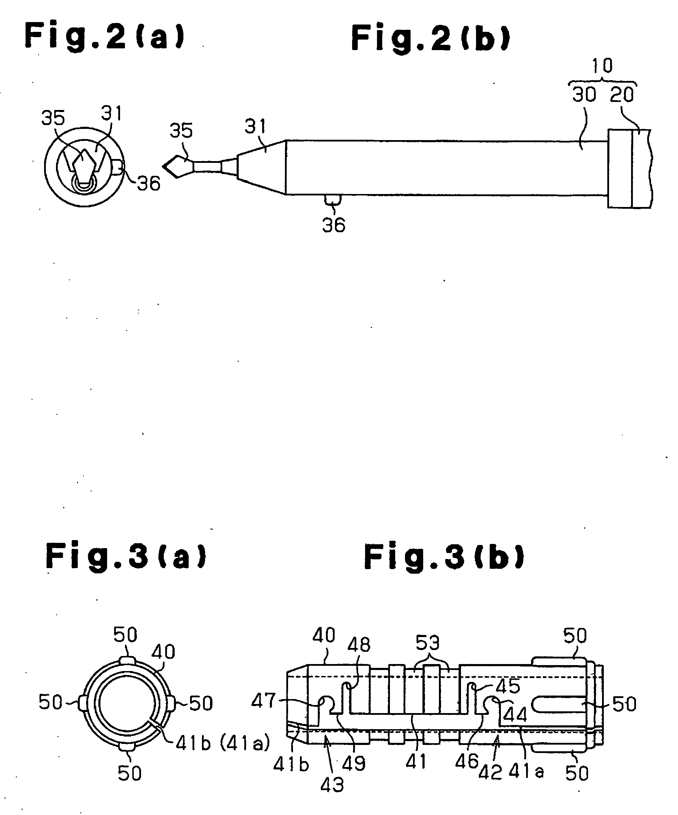

A protective cover 40 is attached to the supporting portion 30. As shown in Fig. 3(a), the protective cover 40 is formed in a cylindrical shape, and the inner peripheral surface of the protective cover 40 is formed so as to have a circular cross section. There is no limitation on a material of the protective cover 40, and the protective cover 40 of the present embodiment is molded with a synthetic resin. As shown in Figs. 1(a) and 1(b), the protective cover 40 is fitted into the outer peripheral surface of the supporting portion 30 so as to slide freely along the axial direction of the supporting portion 30. A slit 41 is formed on the protective cover 40, penetrating through the peripheral wall thereof. The slit 41 extends along the axial direction of the protective cover 40, and the slit 41 extends parallel with the axial direction of the protective cover 40.

A projection 36 is projected on the outer peripheral surface of the distal end portion of the supporting portion 30 so as to be orthogonal to the axial direction of the supporting portion 30. The projection 36 enters and is engaged with the slit 41, and is movable relative to the protective cover 40. The projection 36 is shaped so as to have a circular cross section. The shape of the projection 36 is not limited to a circular cross section. The projection 36 may be shaped so as to have a triangular cross section or a polygonal cross section. On movement of the protective cover 40 along the axial direction thereof, the projection 36 slidingly contacts the inner surface of the slit 41, thereby exhibiting such a guide function to guide the protective cover 40 along the axial direction of the protective cover 40 and also regulating a rotational movement around the axis of the protective cover 40 relative to the supporting portion 30. The projection 36 is given such a length not to project outward from the protective cover 40 via the slit 41. In this way, since the projection 36 is not projected from the slit 41, the projection 36 will not be a hindrance in gripping a surgical scalpel when the surgical scalpel is used.

In the following explanation, of both of the end portions of the slit 41, the end portion adjacent to the grip portion 20 is referred to as a proximal end portion, whereas the end portion spaced away from the grip portion 20 is referred to as a distal end portion. A narrow slit 41a having a width narrower than the dimension of the projection 36 (the diameter in the present embodiment) extends along the axial direction of the protective cover 40 at the proximal end portion of the slit 41. The width of the narrow slit 41a is narrower than that of the slit 41. The narrow slit 41a extends up to the edge of the protective cover 40 and communicates with the outside. Similarly, a narrow slit 41b having the width narrower than the dimension of the projection 36 (the diameter in the present embodiment) extends along the axial direction of the protective cover 40 at the distal end portion of the slit 41. The width of the narrow slit 41b is narrower than that of the slit 41. The narrow slit 41b extends up to the edge of the protective cover 40 and communicates with the outside.

Since these narrow slits 41a, 41b are narrower in width than the dimension of the projection 36, the projection 36 does not move outward from the slit 41. Due to the narrow slits 41a, 41b and the slit 41, the protective cover 40 is expanded by a jig or the like (not illustrated) against the elastic force thereof on assembly of the protective cover 40 and attached to the supporting portion 30. The protective cover 40 is reduced in diameter by the elastic force and fitted into the supporting portion 30 when released from the expansion by the jig or the like.

As shown in Fig. 3(b), locking portions 42, 43 are mounted at the proximal end portion and the distal end portion. The locking portion 42 is made of a notch 44 and a cut groove 45 extending at the proximal end portion in a direction intersecting a direction in which the slit 41 extends (an orthogonal direction in the present embodiment) and an engagement piece 46 formed between them. The engagement piece 46 is elastic due to the cut groove 45, constituting the peripheral part of the notch 44.

The bottom portion of the notch 44 is formed in such a dimension that the projection 36 can enter and be engaged with the notch 44. Also, the width of the inlet of the notch 44 is set to be slightly narrower than the dimension of the projection 36 (the diameter in the present embodiment). When the projection 36 enters and is engaged with the notch 44 via the inlet of the notch 44, the inlet is opened against the elastic force of the engagement piece 46, thereby the projection 36 can be moved into the bottom portion of the notch 44 and engaged with the bottom portion of the notch 44. In a state where the projection 36 is in the notch 44, the protective cover 40 is locked against movement along the axial direction thereof and covers the blade body 35 as shown in Fig. 1(b). In the following explanation, a position at which the blade body 35 is covered by the protective cover 40 is referred to as a covering position.

The locking portion 43 is made of a notch 47 and a cut groove 48 extending at the distal end portion in a direction intersecting direction in which the slit 41 extends (an orthogonal direction in the present embodiment) and an engagement piece 49 formed between them. The engagement piece 49 is elastic due to the cut groove 48, constituting the peripheral part of the notch 47. The bottom portion of the notch 47 is formed in such a dimension that the projection 36 can enter and be engaged with the bottom portion. Also the width of the inlet of the notch 47 is set to be slightly narrower than the dimension of the projection 36. When the projection 36 enters the notch 47 via the inlet of the notch 47, the inlet is opened against the elastic force of the engagement piece 49, thereby the projection 36 can be moved into the bottom portion of the notch 47 and engaged with the bottom portion of the notch 47.

In a state where the projection 36 is in the notch 47, the protective cover 40 is locked against movement along the axial direction thereof and the blade body 35 is exposed, as shown in Fig. 1(a). In the following explanation, a position at which the blade body 35 is exposed is referred to as an exposing position. When the protective cover 40 is placed at the exposing position, the proximal end portion of the protective cover 40 is in contact with the distal end face of the grip portion 20. Manipulating projections 50 are projected at equal intervals on the outer peripheral surface of the proximal end portion of the protective cover 40. A plurality of grooves 53 are formed between the cut groove 45 and the cut groove 48. Each of the grooves 53 is formed along a direction intersecting a direction in which the slit 41 extends and is formed over the entire periphery of the protective cover 40. When the grip portion 20 is held with one hand, the thumb is used to press the groove 53 or the manipulating projections 50, by which the protective cover 40 moves along the axial direction thereof or rotates around the axis.

An explanation will be made of an operation of the above-configured surgical scalpel.

As shown in Fig. 1(b), when the protective cover 40 is placed at the covering position, the projection 36 is engaged with the bottom portion of the notch 44, and the protective cover 40 is locked by the engagement piece 46. In this state, the protective cover 40 does not move even if an attempt is made to move the protective cover 40 along the axial direction thereof, and the blade body 35 is covered by the protective cover 40. Therefore, the surgical scalpel can be safely handed from an assistant to a surgeon during surgical procedures.

When the surgeon uses the scalpel, first, with the grip portion 20 held with one hand, the surgeon puts the thumb of one hand, for example, on the manipulating projection 50, rotating the protective cover 40 around the axis thereof in a direction in which the projection 36 is detached from the engagement piece 46. In this instance, the projection 36 is detached from the notch 44 against the elastic force of the engagement piece 46, making a relative movement to the proximal end portion of the slit 41. In this state, the surgeon uses the thumb to move the protective cover 40 from the covering position to the exposing position.

When the protective cover 40 is in contact with the distal end face of the grip portion 20, the projection 36 is placed at the distal end portion of the slit 41 (refer to Fig. 1(a)). In this state, similarly as described above, with the grip portion 20 held with one hand, the surgeon puts the thumb of one hand, for example, on the manipulating projections 50, rotating the protective cover 40 around the axis thereof in a direction in which the projection 36 enters the notch 47 (a direction intersecting the axial direction). Thereby, the projection 36 enters the notch 44 and locked against the elastic force of the engagement piece 46. As shown in Fig. 1(a), the blade body 35 is exposed from the protective cover 40 by a series of manipulations by the thumb. In this state, since the protective cover 40 is locked, the protective cover 40 does not move even if an attempt is made to move the protective cover 40 along the axial direction.

When the surgeon finishes using the surgical scalpel, he or she conducts the above-described manipulations in a reversed manner, thereby, as shown again in Fig. 1(b), the protective cover 40 is placed at the covering position and locked. Therefore, the surgical scalpel of the present embodiment can be manipulated only by using the thumb of one hand to move the protective cover 40 between the covering position and the exposing position, locking and unlocking it at each position. In locking or unlocking the protective cover 40, the grip portion 20 may be held with the middle finger, the ring finger and the little finger of one hand, or the protective cover 40 may be held between the thumb and the index finger to rotate. In this instance as well, similarly as described above, it is possible to move, lock and unlock the protective cover 40 in a two-stage motion by using only one hand.

The present embodiment provides the following advantages.
(1) In the surgical scalpel of the present embodiment, the handle main body 10 is made of a grip portion 20 formed so as to be held with one hand and a supporting portion 30 coupled integrally with the grip portion 20 to support the protective cover 40 so as to slide on the outer periphery. A projection 36 is formed on the outer periphery of the supporting portion 30, and the protective cover 40 is provided with a pair of locking portions 42 with which the projection 36 makes an elastic engagement respectively when selectively placed at the covering position or at the exposing position.
   As a result, the necessity for mounting a member for locking the protective cover 40 on the handle main body 10 as a separate member is eliminated, the protective cover 40 for protecting the blade body 35 can be easily manipulated by one hand, and the protective cover 40 can also be retained in an exposing state and in a covering state.
(2) In the surgical scalpel of the present embodiment, since the notch 47 extends along a direction intersecting a direction in which the slit 41 extends, a user of the surgical scalpel allows the protective cover 40 to rotate around the axis, thus making it possible to allow the projection 36 to enter the notches 44, 47.
(3) In the surgical scalpel of the present embodiment, the locking portions 42, 43 having the notches 44, 47 extending along a direction intersecting a direction in which the slit 41 extends are located at the proximal end portion and the distal end portion of the slit 41. As a result, only a two-stage motion by the thumb of one hand holding the grip portion 20 is performed, by which the protective cover 40 retained at the covering position is allowed to move easily to the exposing position, and also the protective cover 40 retained at the exposing position is allowed to move easily to the covering position.
(4) In the surgical scalpel of the present embodiment, the locking portions 42, 43 are provided with the notches 44, 47 and the elastic engagement pieces 46, 49 constituting the peripheral parts of the notches 44, 47. As a result, the elastic engagement piece 46 makes an elastic engagement with the projection 36 to retain the projection 36, thus making it possible to retain the protective cover 40 at the covering position or the exposing position.
(5) In the surgical scalpel of the present embodiment, the manipulating projections 50 are formed on the peripheral surface of the protective cover 40. As a result, with the grip portion 20 held with one hand, the thumb of one hand touches the manipulating projections 50, thereby manipulating easily the protective cover 40. In particular, when a thumb is used to perform a two-stage motion, a thumb touches the manipulating projections 50, thereby facilitating the rotation of the protective cover 40.

### (Second Embodiment)

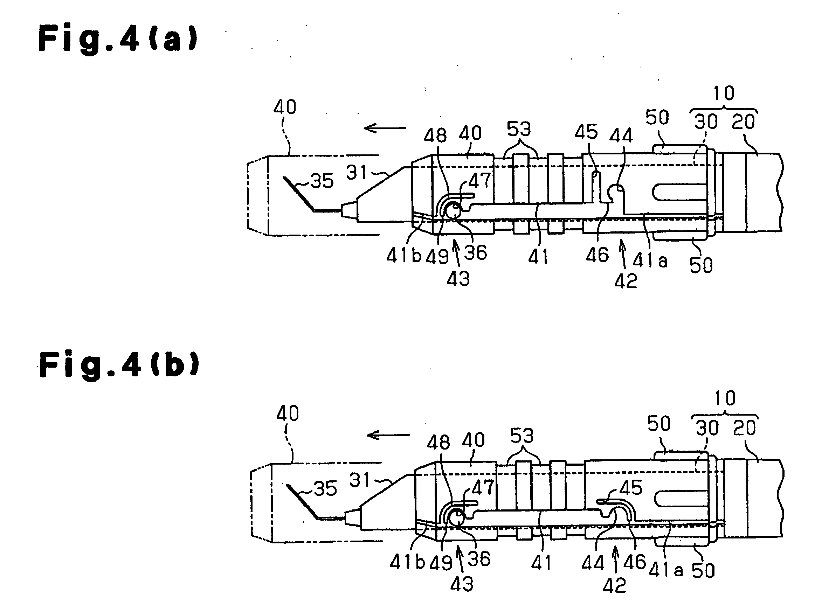

Fig. 4(a) shows the configuration of the surgical scalpel of a second embodiment. The surgical scalpel of the second embodiment is different from the first embodiment only in configuration of the locking portion 43 used in the surgical scalpel of the first embodiment. Therefore, the same components as those of the first embodiment and components corresponding to those of the first embodiment will be given the same reference numerals, an explanation of which will be omitted. Then, an explanation will be mainly made of components different from the first embodiment. In Fig. 4(a), the protective cover 40 indicated by the solid line shows a case where the protective cover 40 is placed at the exposing position, whereas the protective cover 40 indicated by the chain double-dotted line shows a case where the protective cover 40 is placed at the covering position.

In the locking portion 43 of the first embodiment, the notch 47 and the cut groove 48 are mounted in a direction orthogonal to a direction in which the slit 41 extends. However, as shown in Fig. 4(a), in the locking portion 43 of the present embodiment, the cut groove 48 and the notch 47 are formed in such a manner that the engagement piece 49 extends along a direction in which the slit 41 extends. The engagement piece 49 is elastic due to the cut groove 48 and the notch 47. The inlet of the notch 47 communicating with the slit 41 is set to be narrower in width than the slit 41. In other words, the width of the inlet of the notch 47 is set to be narrower than the dimension of the projection 36 (the diameter of the projection 36 in the present embodiment).

When the projection 36 is passed through the inlet of the notch 47, the engagement piece 49 is displaced against the elastic force thereof to open the inlet of the notch 47, thereby the projection 36 can be moved into the bottom portion of the notch 47 and engaged with the bottom portion of the notch 47. Thereafter, the engagement piece 49 is returned to a position shown in Fig. 4(a) by the elastic force of the engagement piece 49, thereby the inlet of the notch 47 is again made narrow to lock the projection 36 at the exposing position.

Since the locking portion 43 is configured as described above, a surgeon uses the thumb of one hand holding the grip portion 20 to allow the protective cover 40 to move along the axial direction of the protective cover 40 and also toward the grip portion 20, thereby locking the protective cover 40 at the exposing position. The surgeon conducts the above-described manipulations in a reversed manner, thereby unlocking the protective cover 40 at the exposing position.

The present embodiment provides the following advantages.
(6) In the surgical scalpel of the present embodiment, the locking portion 42 having the notch 44 formed in a direction intersecting a direction in which the slit 41 extends is mounted only at the proximal end portion of the slit 41. As a result, only a two-stage motion by using the thumb of one hand holding the grip portion 20 is performed, by which the protective cover 40 retained at the covering position is allowed to move easily to the exposing position.
(7) In the surgical scalpel of the present embodiment, a surgeon can move the protective cover 40 from the exposing position to the covering position simply by pressing the protective cover 40 with a finger in the axial direction against the elastic force of the engagement piece 49. This facilitates the operation of the scalpel performed b the surgeon.

### (Third Embodiment)

Fig. 4(b) shows the configuration of the surgical scalpel of a third embodiment. The surgical scalpel of the third embodiment is different from the second embodiment only in the configuration of the locking portion 42 used in the surgical scalpel of the second embodiment. Therefore, the same components as those of the second embodiment and components corresponding to those of the second embodiment will be given the same reference numerals, an explanation of which will be omitted. Then, an explanation will be mainly made of components different from the second embodiment. In Fig. 4(b), the protective cover 40 indicated by the solid line shows a case where the protective cover 40 is placed at the exposing position, whereas the protective cover 40 indicated by the chain double-dotted line shows a case where the protective cover 40 is placed at the covering position.

In the locking portion 42 of the first embodiment, the notch 44 and the cut groove 45 are formed in a direction orthogonal to a direction in which the slit 41 extends. However, as shown in Fig. 4(b), at the locking portion 42 of the present embodiment, the cut groove 45 and the notch 44 are formed in such a manner that the engagement piece 46 extends along a direction in which the slit 41 extends. The engagement piece 46 is elastic due to the cut groove 45 and the notch 44. The inlet of the notch 44 communicating with the slit 41 is set to be narrower in width than the slit 41. In other words, the width of the inlet of the notch 44 is set to be narrower than the dimension of the projection 36 (the diameter of the projection 36 in the present embodiment).

When the projection 36 is passed through the inlet of the notch 44, the engagement piece 46 is displaced against the elastic force thereof to open the inlet of the notch 44, thereby the projection 36 can be moved into the bottom portion of the notch 44 and engaged with the bottom portion of the notch 44. Thereafter, the engagement piece 46 is returned to a position shown in Fig. 4(b) by the elastic force of the engagement piece 46, thereby the inlet of the notch 44 is again made narrow to lock the projection 36 at the covering position.

Since the locking portion 42 is configured as described above, a surgeon uses the thumb of one hand holding the grip portion 20 to allow the protective cover 40 to move along the axial direction of the protective cover 40 and also to be spaced away from the grip portion 20, thereby locking the protective cover 40 at the covering position. The surgeon performs the above-described manipulations in a reversed manner, thereby unlocking the protective cover 40 at the covering position.

The present embodiment provides the following advantages.
(8) In the surgical scalpel of the present embodiment, a surgeon can lock and unlock the protective cover 40 at the covering position and at the exposing position simply by pressing the protective cover 40 in the axial direction against the elastic force of the engagement pieces 46, 49.

### (Fourth embodiment)

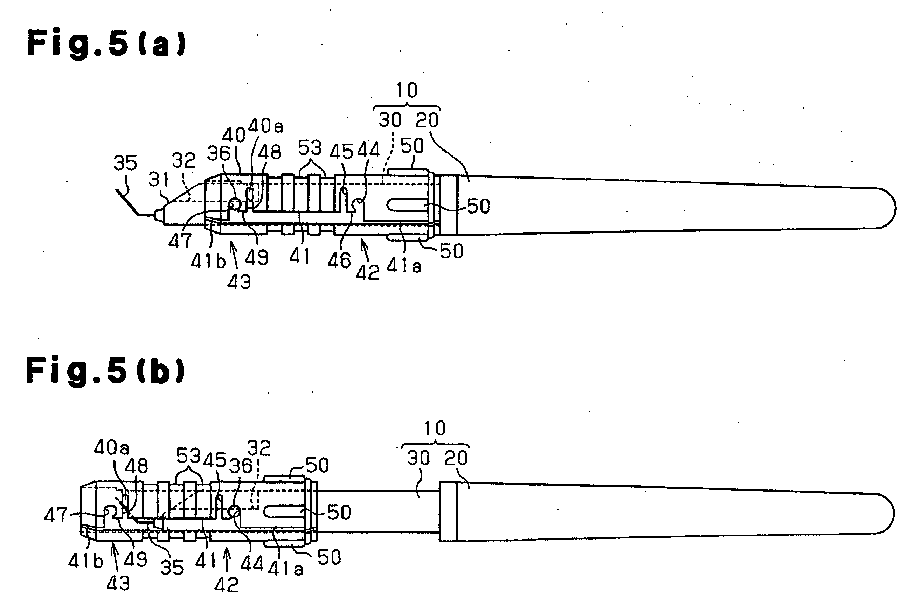

Hereinafter, an explanation will be made of a fourth embodiment where the medical edged tool of the present invention is applied to a scalpel for ophthalmic surgery (hereinafter, simply referred to as surgical scalpel) by referring to Figs. 5 to 9. In the fourth embodiment, the same components as those of the first embodiment and components corresponding to those of the first embodiment will be given the same references, an explanation of which will be omitted. Then, an explanation will be mainly made of components different from the first embodiment.

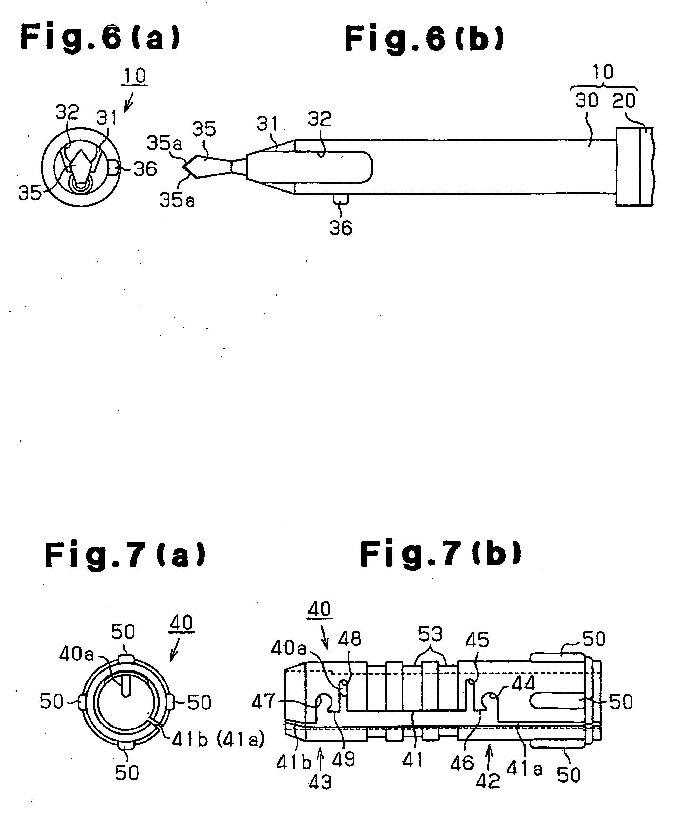

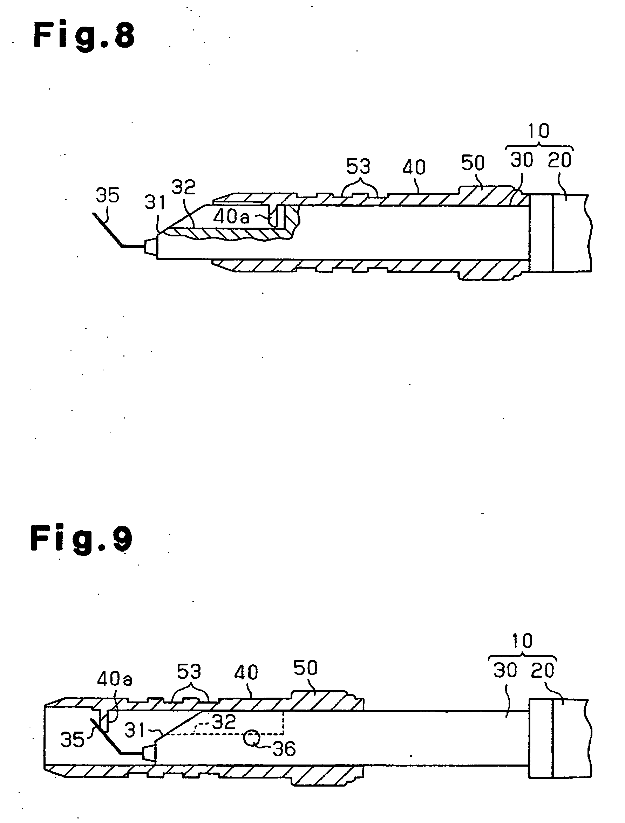

As shown in Figs. 5(a) and 5(b), the grip portion 20 is set to be gradually smaller in outer diameter toward the proximal end of the grip portion 20 (the right end in Fig. 5(a)). As shown in Figs. 5(a) to 6(b), the blade body 35 is a bent-type blade body which is bent at an angle less than 90 degrees toward the inclined surface 31 between the central part and the proximal end portion. The blade body 35 is bent so as to have an inner surface and an outer surface. As shown in Fig. 6(b), the blade body 35 is formed angularly at the distal end portion and provided with a cutting edge 35a at both side edges. In other words, the blade body 35 has a spear-like blade. As shown in Fig. 8, an allowance groove 32 is formed as an allowance portion on the outer peripheral surface of the supporting portion 30. The allowance groove 32 is formed so as to extend from the inclined surface 31 to the grip portion 20 and also extend along the axial direction of the supporting portion 30.

As shown in Figs. 5(a), 5(b), 7(a), and 7(b), a deflection restraining projection 40a as a deflection restraining member is formed on the inner wall surface of the distal end portion corresponding to the allowance groove 32 of the supporting portion 30 on the protective cover 40. The deflection restraining projection 40a is formed in a rod shape projecting to the radial direction of the protective cover 40. As shown in Fig. 9, the deflection restraining projection 40a is formed so as to be in contact with the inner surface of the blade body 35 but not to be in contact with the cutting edge, when the protective cover 40 is placed at the covering position. As shown in Fig. 8, the deflection restraining projection 40a is formed so as to enter an allowance groove 32 and be engaged with an allowance groove 30 when the protective cover 40 is placed at the exposing position. The allowance groove 32 is given such a width that the deflection restraining projection 40a is not in contact with the wall surface of the allowance groove 32, even if the protective cover 40 is allowed to rotate when locked or unlocked at the exposing position.

An explanation will be made of actions of the above-configured surgical scalpel.

As shown in Figs. 5(b) and 9, when the protective cover 40 is placed at the covering position, the blade body 35 is covered by the protective cover 40. In this state, the deflection restraining projection 40a is in contact with the central inner surface of the distal end portion of the blade body 35. Therefore, when load is applied to the blade body 35, for example, when a surgical scalpel is being transported or dropped, the blade body 35 does not deflect to the bending direction thereof. Further, in this state, it is possible to hand over the surgical scalpel from an assistant to a surgeon safely during surgical procedures.

When a surgeon uses the scalpel, first, with the grip portion 20 held with one hand, the surgeon puts the thumb of one hand, for example, on the manipulating projections 50, rotating the protective cover 40 around the axis thereof in a direction in which the projection 36 is detached from the engagement piece 46. In this instance, the projection 36 is detached from the notch 44 against the elastic force of the engagement piece 46, making a relative movement to the proximal end portion of the slit 41. In this state, the surgeon uses a thumb to move the protective cover 40 from the covering position to the exposing position. At this time, the deflection restraining projection 40a enters and is engaged with the allowance groove 32.

When the protective cover 40 is in contact with the distal end face of the grip portion 20, the projection 36 is placed at the distal end portion of the slit 41 (refer to Fig. 5(a)). In this state, similarly as described above, with the grip portion 20 held with one hand, the surgeon puts the thumb of one hand, for example, on the manipulating projections 50, rotating the protective cover 40 around the axis thereof in a direction in which the projection 36 enters the notch 47 (a direction intersecting the axial direction). Thereby, the projection 36 enters the notch 44 and is locked against the elastic force of the engagement piece 46. As shown in Fig. 5(a), the blade body 35 is exposed from the protective cover 40 by a series of manipulations by the thumb. In this state, since the protective cover 40 is locked, the protective cover 40 does not move even if an attempt is made to move the protective cover 40 along the axial direction.

When a surgeon finishes using the surgical scalpel, he or she performs the above-described manipulations in a reversed manner, thereby, as shown again in Fig. 5(b), the protective cover 40 is placed at the covering position and locked. In this instance, as shown in Fig. 9, the deflection restraining projection 40a is again in contact with the blade body 35.

The present embodiment provides the following advantages.
(9) In the surgical scalpel of the present embodiment, the deflection restraining projection 40a which is in contact with the blade body 35 when the protective cover 40 is placed at the covering position is provided on the inner wall surface of the protective cover 40. As a result, even when load is applied to the blade body 35 such as when the surgical scalpel is being transported or is dropped, the deflection restraining projection 40a of the protective cover 40 is in contact with the blade body 35, making it possible to prevent the deflecting of the blade body 35, thus keeping the tip of the cutting edge undamaged.
(10) In the scalpel for ophthalmic surgery of the present embodiment, the blade body 35 is a bent-type blade body in which the deflection restraining projection 40a is allowed to be in contact with the inner surface of the blade body 35 when the protective cover 40 is placed at the covering position. As a result, the above-described advantages of (9) can be provided for a surgical scalpel having the bent-type blade body.
(11) In the scalpel for ophthalmic surgery of the present embodiment, the allowance groove 32 for allowing the deflection restraining projection 40a to move when protective cover 40 is placed at the exposing position is formed at the outer periphery of the handle main body 10. As a result, in the present embodiment, when the protective cover 40 moves to the exposing position, the deflection restraining projection 40a is accommodated into the allowance groove 32 of the handle main body 10. Thus, the handle main body 10 is unobstructed, and there is no failure in manipulating the protective cover 40 so as to move.

When a scalpel is used, for example, as a medical edged tool, the scalpel is important in cutting proficiently and provided with a sharp tip of cutting edge. Since there are concerns that the tip of cutting edge may accidentally damage the hands of a surgeon or the hands of other medical personnel when a scalpel is handed over from an assistant to a surgeon during surgical procedures or in preparation for surgical procedures, various types of scalpels with a protective cover have been proposed.

The scalpel disclosed in the U.S. Patent No. 5309641 is provided at the distal end of a handle with a knife-type blade body. A protective cover is mounted so as to slide freely along the longitudinal direction of the handle. When the scalpel is not in use, the blade body is covered by the protective cover, and when the scalpel is in use, the protective cover is moved to expose the blade body.

The scalpel disclosed in U.S. Patent No. 6254621 is provided with a knife-type blade body which is accommodated into a handle so as to slide freely. When the scalpel is not in use, the blade body is accommodated into the handle, and when the scalpel is in use, the blade body is projected from the handle.

The scalpels disclosed in U.S. Patent No. 6569175 and U.S. Design Patent No. 504513 are each provided with a bent-type blade body. In these scalpels, a protective cover is mounted so as to slide freely along the longitudinal direction of a handle. When the scalpel is not in use, the blade body is covered by the protective cover, and when the scalpel is in use, the protective cover is moved to expose the blade body. A bulging portion is formed on the protective cover disclosed in U.S. Design Patent No. 504513 so as to accommodate a bent blade body, and when the protective cover is moved to a position at which the blade body is covered, the blade body is accommodated into the bulging portion. Further, in these scalpels, when they are not in use, for example during transportation, the blade body is covered by the protective cover.

In these medical edged tools, the blade body is fixed to a handle, while a cutting part of the blade body is kept unfixed. In general, medical edged tools are approximately 0.15 mm to 0.20 mm in thickness, and the blade body is easily subjected to deflecting when medical edged tools are being transported or they are dropped accidentally. When the blade body deflects in a state where the blade body is covered by the protective cover, the blade body may be in contact with the inner wall of the protective cover to result in a damaged tip of the cutting edge of the blade body.

In the bent-type scalpel disclosed in U.S. Patent No. 6569175, in particular, the blade body is easily in contact with the protective cover. On the other hand, in the scalpel disclosed in U.S. Design Patent No. 504513, a bulging portion for accommodating the blade body is provided. Therefore, as compared with the scalpel disclosed in U.S. Patent No. 6569175, the tip of the cutting edge of the blade body is less likely to be damaged. However, in the scalpel disclosed in U.S. Design Patent No. 504513, a bulging portion is provided in the vicinity of the blade body. Therefore, even in a state where the protective cover moves toward a handle to expose the blade body when the scalpel is in use, the bulging of the bulging portion is adjacent to the blade body and a user finds it difficult to visually observe the tip of cutting edge of the blade body.

### (Fifth Embodiment)

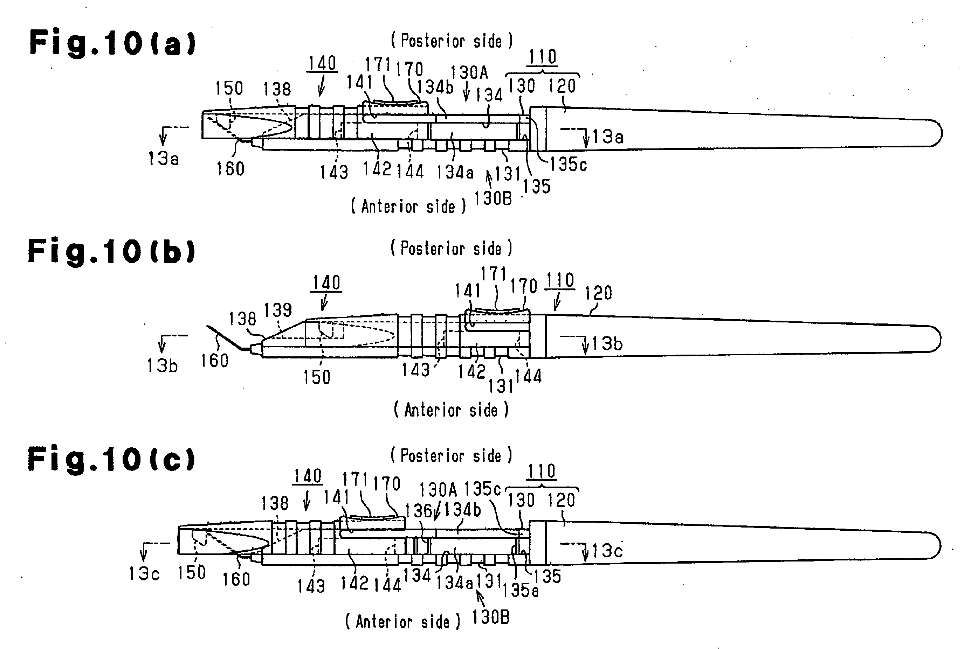

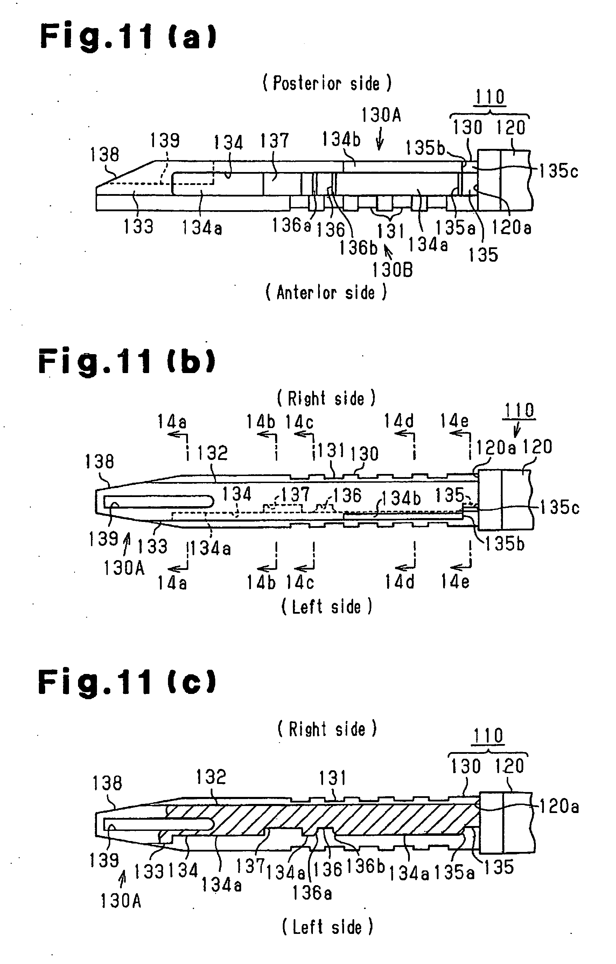

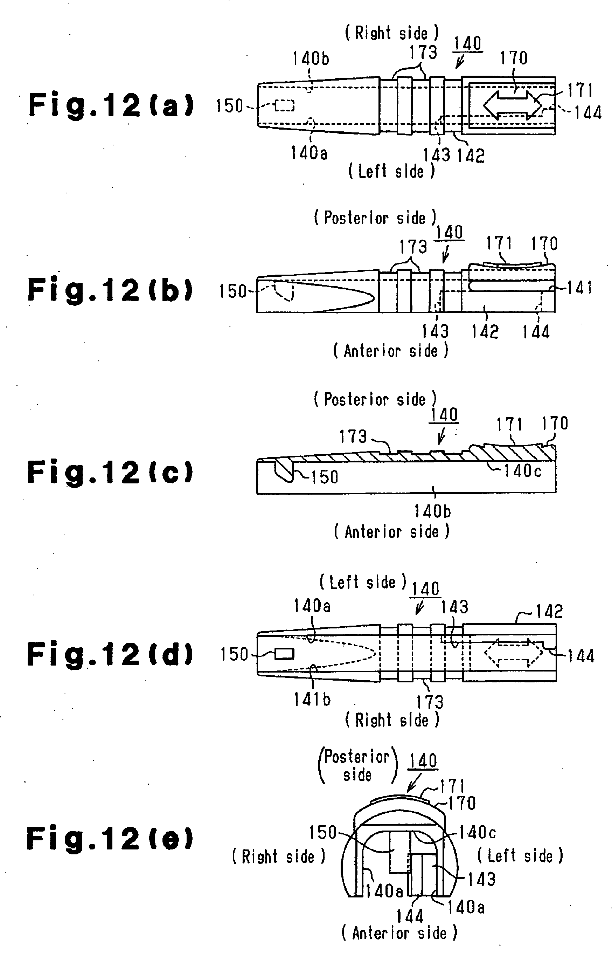

Figs. 10 to 14 show the configuration of the surgical scalpel of a fifth embodiment. In Figs. 10, 11(a), 12(b), 12(c), and 12(e) and Figs. 14(a) to 14(e), the upper part of each of the drawings corresponds to the posterior side of the member shown in each of the drawings, whereas the lower part corresponds to the anterior side of the member. In Figs. 11(b), 11(c), 12(a), and 12(d), and Figs. 13(a) to 13(c), the upper part of each of the drawings corresponds to the right side of a member shown in each of the drawings, whereas the lower part corresponds to the left side of the member. The left side of Fig. 12(e) corresponds to the right side of the member shown in the drawing, whereas the right side thereof corresponds to the left side of the member.

As shown in Figs. 10(a) and 10(b), a handle main body 110 of the surgical scalpel is provided with a grip portion 120 and a supporting portion 130 mounted at the distal end of the grip portion 120 coaxially with the grip portion 120. The grip portion 120 is formed in the same shape as the grip portion 20 of the fourth embodiment and made with the same material.

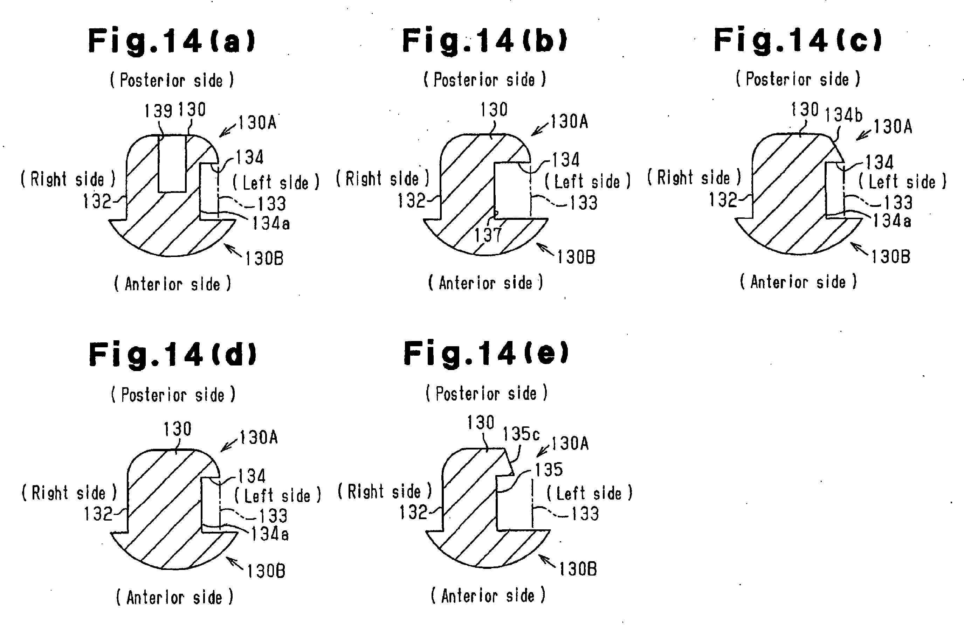

As shown in Figs. 11(a) to 11(c), a supporting portion 130 is formed in a columnar shape and made of a slide rail portion 130A having an substantially rectangular cross section and an anterior portion 130B positioned below the slide rail portion 130A and formed integrally with the slide rail portion 130A. As shown in Figs. 14(a) to 14(e), the peripheral surface of the anterior portion 130B has a circular-arc cross section. In Figs. 11(a) to 11(c), the blade body to be described below is omitted for the convenience of explanation. The peripheral surface of the anterior portion 130B is formed so as to be flush with the peripheral surface of the grip portion 120. A plurality of grooves 131 are formed on the peripheral surface of the proximal end portion at the anterior portion 130B.

A pair of sliding contact surfaces 132, 133 are formed on the right and left side surfaces of the slide rail portion 130A so as to extend parallel with each other along the axial direction of the supporting portion 130. As shown in Figs. 11(b) and 11(c), the sliding contact surface 132 is a flat surface extending from the distal end face 120a of the grip portion 120 along the axial direction of the grip portion 120. In contrast, the sliding contact surface 133 is provided with a slide groove 134 over the entire remaining portion excluding the distal end portion thereof. As shown in Fig 11(c), the distal end face of the grip portion 120 constitutes the end face of the slide groove 134. As shown in Figs. 11(b) and 11(c), the bottom portion 134a of the slide groove 134 has a flat surface extending along the axial direction of the supporting portion 130 and the flat surface extends parallel with the sliding contact surface 132.

A first engaging recess 135 having a rectangular cross section is formed at the bottom portion 134a. The distal end face 120a of the grip portion 120 constitutes the wall surface of the first engaging recess 135. A guide surface 135a is formed at the edge facing the distal end face 120a in an opening of the first engaging recess 135. The guide surface 135a is inclined to the distal end face 120a from the opening of the first engaging recess 135 toward the inside of the recess 135. The guide surface 135a is coupled to the bottom portion 134a of the slide groove 134.

A second engaging recess 136 having the same shape of the cross section and the same dimension as the first engaging recess 135 is formed at the center of the bottom portion 134a in the longitudinal direction. At an opening of the second engaging recess 136, guide surfaces 136a, 136b are formed respectively on both edges of the bottom portion 134a in the longitudinal direction. These guide surfaces 136a, 136b are inclined so as to come closer to each other from the opening of the second engaging recess 136 toward the inside of the recess 136. These guide surfaces 136a, 136b are respectively coupled to the bottom portion 134a of the slide groove 134. A third engaging recess 137 is formed at a site closer to the distal end of the slide groove 134 than the second engaging recess 136 at the bottom portion 134a. The length of the bottom portion 134a of the third engaging recess 137 in the longitudinal direction, that is, the length of the supporting portion 130 along the axial direction is set to be longer than that of the second engaging recess 137 in the same direction.

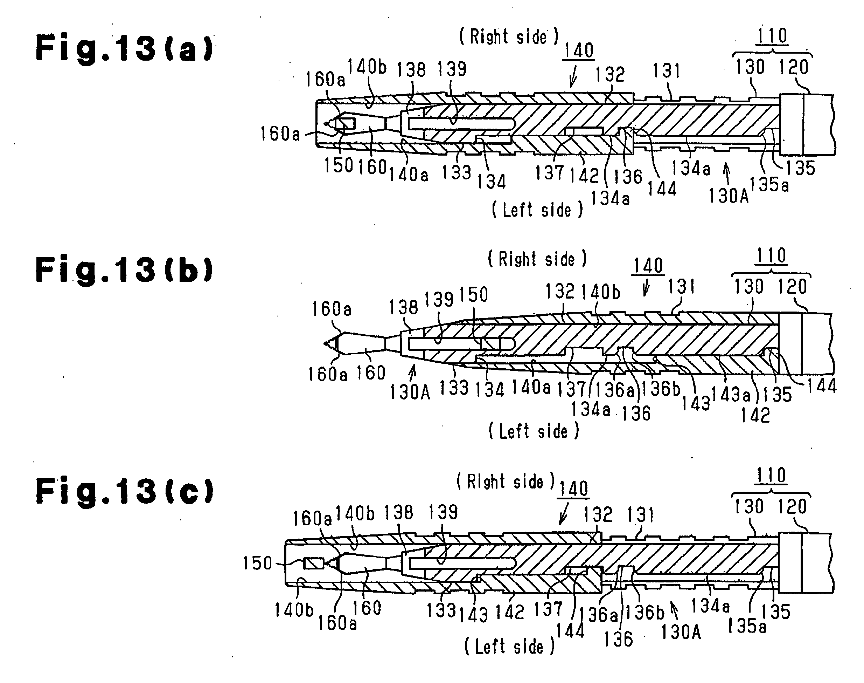

An inclined surface 138 is formed at the distal end of the slide rail portion 130A. A blade body 160 is attached at a site made eccentric from the supporting portion 130 on the distal end face of the supporting portion 130. The blade body 160 is a bent-type blade body which is bent at less than 90° toward the inclined surface 138 between the central part and the proximal end portion. The blade body 160 is bent so as to have an inner surface and an outer surface. As shown in Fig. 13(b), the blade body 160 is formed angularly at the distal end portion and provided with a cutting edge 160a at both side edges.

As shown in Figs. 11(a) to 11(c), an allowance groove 139 as an allowance portion is formed on the peripheral surface on the posterior side of the slide rail portion 130A in such a manner as to extend from the inclined surface 138 to the grip portion 120 and also parallel with the axis of the supporting portion 130.

As shown in Figs. 10(a) to 10(c) and Figs. 13(a) to 13(c), a protective cover 140 is attached to the slide rail portion 130A of the supporting portion 130. As shown in Fig. 12 (e), the protective cover 140 is formed substantially in a semi-cylindrical shape. The inner peripheral surface of the protective cover 140 is made of a left inner surface 140a, a right inner surface 140b, and an inner upper wall surface 140c for connecting the inner surface 140a with the inner surface 140b, and the protective cover 140 is opened at the anterior side.

The left inner surface 140a, the right inner surface 140b and the inner upper wall surface 140c are slidingly in contact with the sliding contact surfaces 133, 132 of the slide rail portion 130A and the peripheral surface on the posterior side, by which the protective cover 140 is fitted into the slide rail portion 130A so as to slide freely along the axial direction of the slide rail portion 130A. There is no limitation on a material of the protective cover 140, and the protective cover 140 of the present embodiment is made with, for example, a synthetic resin.

As shown in Fig. 12(b), a notch 141 is formed along the axial direction of the protective cover 140 at the proximal end portion of a side corresponding to the slide groove 134 on the protective cover 140. The notch 141 forms an elastic engagement piece 142 on the protective cover 140. A step 143 is formed at the engagement piece 142 and a region covering from the engagement piece 142 to the distal end of the protective cover 140 on the inner surface of the side corresponding to the slide groove 134 of the protective cover 140. The step 143 is fitted so as to slide freely on the slide groove 134. A projection 144 is formed on the inner surface of the distal end of the engagement piece 142. The projection 144 is formed so as to be engaged with the first engaging recess 135, the second engaging recess 136 and the third engaging recess 137 due to the elastic force of the engagement piece 142.

When the projection 144 is engaged with the second engaging recess 136, the protective cover 140 is positioned so as to cover the blade body 160 as shown in Fig. 13(a). In the following explanation, this position is referred to as a covering position. When the protective cover 140 is pressed to the blade body 160 or-the grip portion 120 in a state where the protective cover 140 is placed at this position, the projection 144 is guided by guide surfaces 136a, 136b of the second engaging recess 136 and removed from the second engaging recess 136.

When the projection 144 is engaged with the first engaging recess 135, the protective cover 140 is placed so as to expose the blade body 160 as shown in Fig. 13(b). In the following explanation, this position is referred to as an exposing position. When the protective cover 140 is pressed to the blade body 160 from the first engaging recess 135 in a state where the protective cover 140 is placed at this position, the projection 144 is guided by the guide surface 135a of the first engaging recess 135 and removed from the first engaging recess 135.

When the projection 144 is engaged with the third engaging recess 137, the protective cover 140 keeps the blade body 160 covered as shown in Fig. 13(c). In this state, even if the protective cover 140 is pressed from the third engaging recess 137 to the blade body 160, the step 143 is engaged with the end portion of the slide groove 134, thereby making the protective cover 140 immovable after an allowed movement of a play part is complete. Further, in this state, even if the protective cover 140 is pressed from the third engaging recess 137 to the grip portion 120, the projection 144 is engaged with the end portion of the third engaging recess 137, thereby making the protective cover 140 immovable. As a result, the projection 144 cannot be removed from the third engaging recess 137, and the protective cover 140 is made immovable from a position shown in Fig. 13(c). In the following explanation, a position at which the projection 144 is engaged with the third engaging recess 137 is referred to as a lock position. As described so far, in the present embodiment, the lock means is made of the end portion of the slide groove 134 which can be engaged with the slide step 143 and the third engaging recess 137. The lock means locks the movement of the protective cover 140 so as not to be unlocked in a state where the protective cover 140 covers the blade body 160. The lock position referred to in the present embodiment includes a range at which the play part of the protective cover 140 is allowed to move as described above. However, when the protective cover 140 is engaged with the third engaging recess 137, the movement of the play part may be omitted. In this instance, the lock position does not include the above range but includes only a position at which the projection 144 is engaged with the third engaging recess 137.

A deflection restraining projection 150 as a deflection restraining member is formed on the inner upper wall surface 140c at the distal end portion of the protective cover 140 corresponding to the allowance groove 139 of the supporting portion 130. The deflection restraining projection 150 is formed in a rod shape so as to project radially to the protective cover 140. The projection 150 may be made as a member separate from the protective cover 140 and may be assembled to the protective cover 140 or formed integrally with the protective cover 140 by a joining method such as adhesion or welding. As shown in Fig. 10(a), the deflection restraining projection 150 is formed so as to be in contact with the inner surface at the center of the distal end portion of the blade body 160 when the protective cover 140 is placed at the covering position.

As shown in Figs. 10(b) and 13(b), the deflection restraining projection 150 is formed so as to enter and be engaged with the allowance groove 139 when the protective cover 140 is placed at the exposing position. As shown in Figs. 10(c) and 13(c), the deflection restraining projection 150 is placed at a position exceeding the distal end of the blade body 160 when the protective cover 140 is placed at the lock position.

As shown in Figs. 11(a), 11(b) and 14(e), an engaging port 135b is formed on a wall portion of the posterior side of the first engaging recess 135 at the slide rail portion 130A. A guide surface 135c having an oblique portion is formed on the peripheral surface at the posterior side of the slide rail portion 130A corresponding to the engaging port 135b.

As shown in Fig. 10(b), when the protective cover 140 is placed at the exposing position, a guide surface 134b having an oblique portion shown in Figs. 11(a), 11b), and 14(c) is formed on the peripheral surface at the posterior side of the slide rail portion 130A corresponding to the step 143.

As shown in Figs. 14(a), 14(b), and 14 (d), a corner connecting the sliding contact surface 132 with the upper surface of the posterior side is formed so as to have a circular-arc cross section on the peripheral surface of the slide rail portion 130A. As shown in Figs. 14(a), 14(b), and 14(d), a corner connecting the left side surface excluding the guide surfaces 134b, 135c with the upper surface of the posterior side is formed so as to have a circular-arc cross section on the peripheral surface of the slide rail portion 130A.

The protective cover 140 is fitted to the supporting portion 130 by being pressed against the slide rail portion 130A from the posterior side in a state where the projection 144 is placed so as to correspond to the engaging port 135b. In this instance, when the step 143 of the protective cover 140 and the projection 144 are pressed to the guide surfaces 134b, 135c, the engagement piece 142 is opened against the elasticity thereof. Then, the projection 144 of the engagement piece 142 enters the first engaging recess 135 via the guide surface 135c, and the step 143 also enters the slide groove 134, by which the engagement piece 142 enters and is engaged with the slide groove 134. In this instance, the projection 144 is engaged with the first engaging recess 135 and the step 143 is also engaged with the slide groove 134.

A manipulating portion 170 having on the upper surface of the posterior side a display portion 171 indicating a pressing/manipulating direction is mounted at the proximal end portion of the protective cover 140. A plurality of grooves 173 are formed at the distal end from the manipulating portion 170 on the protective cover 140.

An explanation will be made of an operation of the above-configured surgical scalpel.

As shown in Figs. 10 (a) and 13(a), when the protective cover 140 is placed at the covering position, the projection 144 is engaged with the second engaging recess 136. In this state, since the protective cover 140 is locked by the engagement piece 142, a small force does not move the protective cover 140 even if an attempt is made to move the protective cover 140 to the axial direction thereof, and the blade body 160 is covered by the protective cover 140. In this state, as shown in Fig. 10(a), the deflection restraining projection 150 is in contact with the inner surface at the center of the distal end portion of the blade body 160. Therefore, even when load is applied to the blade body 160, for example, when a surgical scalpel is being transported or dropped, the blade body 160 does not deflect. Further, in this state, the surgical scalpel can be safely handed over from an assistant to a surgeon during surgical procedures.

When a surgeon uses the scalpel, first, with the grip portion 120 held with one hand, the surgeon puts the thumb of one hand on the manipulating portion 170, moving the protective cover 140 the covering position to the exposing position. In this instance, the projection 144 is guided by the guide surface 136b against the elastic force of the engagement piece 142 and removed from the second engaging recess 136, thereby moving along the bottom portion 134a. Further, the deflection restraining projection 150 enters and is engaged with the allowance groove 139.

Then, the protective cover 140 is in contact with the distal end face of the grip portion 120, thereby the projection 144 enters the first engaging recess 135 due to the elastic force of the engagement piece 142 and locked. As shown in Figs. 10(b) and 13(b), the blade body 160 is kept exposed from the protective cover 140 by a series of manipulations by the thumb. In this state, since the protective cover 140 is locked, a small force does not move the protective cover 140 even if an attempt is made to move the protective cover 140 along the axial direction.

When the surgeon finishes using the surgical scalpel, with the grip portion 120 gripped by one hand, he or she puts a thumb on the manipulating portion 170, moving the protective cover 140 to from an exposing position to a lock position. With this manipulation, as shown in Fig. 10(c), the protective cover 140 moves to the lock position. In this instance, the projection 144 is guided by the guide surface 136b and temporarily accommodated into the second engaging recess 136. However, the projection 144 is guided by the guide surface 136a (refer to Fig. 13 (c)), is removed from the second engaging recess 136, enters the third engaging recess 137 via the bottom portion 134a due to the elastic force of the engagement piece 142, and is engaged with the third engaging recess 137.

When the protective cover 140 moves from the exposing position to the lock position, the deflection restraining projection 150 is temporarily in contact with the blade body 160. However, the protective cover 140 further moves against the elastic force of the blade body 160. Then, the blade body 160 undergoes an elastic deformation, thereby the deflection restraining projection 150 is allowed to move, and placed at a position exceeding the blade body 160 as shown in Figs. 10(c) and 13(c). When the protective cover 140 is placed at the lock position, the projection 144 cannot be removed from the third engaging recess 137 and restrained from moving to the exposing position by engagement of the blade body 160 with the deflection restraining projection 150. Therefore, the protective cover 140 does not move to the covering position or the exposing position.

The present embodiment provides the following advantages, in addition to the advantages similar to (9), (10), and (11) described previously in the fourth embodiment.
(12) In the surgical scalpel of the present embodiment, the lock means is made of the end portion of the slide groove 134 which can be engaged with the slide step 143 and the third engaging recess 137. Then, the lock means locks the movement of the protective cover 140 so as not to be unlocked in a state where the blade body 160 is covered by the protective cover 140. As a result, the protective cover 140 locked by the lock means so as not to be unlocked is not allowed to move, and the blade body is kept covered so that the medical edged tool can be handled safely.
(13) In the present embodiment, the end portion of the slide groove 134, which can be engaged with the slide step 143, and the third engaging recess 137 lock the protective cover 140 when the protective cover 140 moves from the covering position to a direction opposite to the exposing position. As a result, the protective cover can be easily locked against movement only by linearly moving the protective cover 140 to from the covering position.
(14) In the present embodiment, when the protective cover 140 is locked, the deflection restraining projection 150 as a deflection restraining member is placed at a position exceeding the distal end of the blade body 160. As a result, the deflection restraining projection 150 prevents foreign matter or others from entering the protective cover 140.

The previously described embodiments may be modified as follows.

In the first and second embodiments, the supporting portion 30 is formed so as to have a circular cross section, excluding the distal end portion thereof. However, the supporting portion 30 may be formed in a cylindrical shape as a whole. The cross section of the supporting portion 30 is not limited to a circular shape but the supporting portion 30 may be formed so as to have an oval cross section. In the first and second embodiments, the supporting portion 30 may only have such a cross sectional shape that the protective cover 40 is allowed to rotate around the axis thereof.

In each of the embodiments, the cross sectional shape of the grip portion 20 may be changed as long as it allows the grip portion 20 to be held by one hand. For example, the cross sectional shape may be a triangular shape, an oval shape, a polygonal shape and a spatula-like flat shape, in addition to a circular shape.

In the first and second embodiments, the notch 44 is formed along an orthogonal direction as a direction intersecting the axial direction of the protective cover 40. However, it may be formed along a direction which is inclined relative to the axial direction of the protective cover 40.

In the first to third embodiments, the manipulating projection 50 is mounted on the outer peripheral surface at the proximal end portion of the protective cover 40. However, in place of the manipulating projection 50, a knurling may be formed on the outer peripheral surface at the proximal end portion of the protective cover 40.

In the first to third embodiments, the present invention is applied to surgical scalpels. The surgical scalpel includes a scalpel for ophthalmic surgery and a microsurgical scalpel for neurosurgical operation. The present invention may be applied to, for example, a large-sized scalpel used in anatomy or the like and an edged tool (trimming knife) for cutting out pathological specimens. Further, the present invention may be applied to a utility knife used in various crafts, for example.

In the fourth embodiment, as shown in Fig. 9, the deflection restraining projection 40a is allowed to be in contact with the inner surface at the center of the distal end portion of the blade body 35 when the protective cover 40 is placed at the covering position. In place of this configuration, the following configuration may be adopted. That is, since the blade body 35 is a bent-type blade body, the blade body 35 is likely to deflect in a bending direction. Therefore, when the protective cover 40 is placed at the covering position, the deflection restraining projection 40a may be formed so as to be located at a position spaced away from the cutting edge 35 and also within the trace of the movement when the blade body 35 deflects in a direction in which the cutting edge 35a deflects (specifically, in a bending direction of the blade body 35). In this configuration, when load is applied to the blade body 35 in a state where the protective cover 40 is placed at the covering position, the blade body 35 is in contact with the deflection restraining projection 40a, thus restraining the restraining projection 40a from deflecting to a great extent although the blade body 35 deflects to some extent.

In the fourth embodiment, the allowance groove 32 is formed as an allowance portion for receiving the deflection restraining projection 40a. However, in the fourth embodiment, the peripheral surface of the supporting portion 30 may be cut up to a part corresponding to the groove bottom of the allowance groove 32, thereby forming a step as the allowance portion on the supporting portion 30. In this configuration as well, on movement of the protective cover 40 to the exposing position, the step acts in such a manner that the deflection restraining projection 40a does not collide with the handle main body 10, thereby allowing the protective cover 40 to move.

The deflection restraining members (deflection restraining projections) of the fourth and fifth embodiments are formed in a rod shape. However, there is no limitation on the shape of the deflection restraining members, and the deflection restraining members may be formed in a conical shape, a truncated conical shape, a square rod shape, or a circular columnar shape.

In the fourth and fifth embodiments, the present invention is applied to a scalpel for ophthalmic surgery having a bent-type blade body as a medical edged tool. The present invention may be applied to a medical edged tool having a blade body other than the bent-type blade body. Medical edged tools include a microsurgical scalpel for for neurosurgical operation other than the scalpel for ophthalmic surgery. The present invention may be applied to, for example, a large-sized scalpel used in anatomy or the like and an edged tool (trimming knife) for cutting out pathological specimens.

In the fourth and fifth embodiments, the deflection restraining projections 40a, 150 are allowed to be in contact with the inner surface at the center of the distal end portion of the blade bodies 35, 160. However, they may be in contact with the proximal end portions of the blade bodies 35, 160 or may be configured so as to be in contact with the proximal end portions of the blade bodies 35, 160 by being positioned within a trace of the movement when the blade bodies 35, 160 deflect.

The configurations of the first to third embodiments may be applied to the medical edged tools of the fourth embodiment and fifth embodiment.

## Claims

1. An edged tool with a protective cover (40), comprising a handle main body (10) having a blade body (35) at a distal end, wherein the protective cover (40) is placed on an outer periphery of the handle main body (10), so as to slide freely along an axial direction of the handle main body (10) between a position at which the protective cover (40) covers the blade body (35) and a position at which the blade body (35) is exposed,
wherein the handle main body (10) includes a grip portion (20) formed so as to be held by one hand and a supporting portion (30) integrally coupled to the grip portion (20) to support the protective cover (40) so as to allow the protective cover (40) to slide on the outer periphery,
wherein a projection (36) is formed on the outer periphery of the supporting portion (30),
wherein a pair of locking portions (42, 43) are formed on the protective cover (40), wherein the projection (36) makes an elastic engagement with the locking portions (42, 43) when the protective cover (40) is selectively placed at the covering position and the exposing position, the edged tool being **characterized in that**
a slit (41) extending along the axial direction of the protective cover (40) is formed in the protective cover (40), the projection (36) enters and is engaged with the slit (41), so as to be movable relative to the slit (41),
wherein the pair of locking portions (42, 43) are formed on both ends of the slit (41),
wherein at least one of the locking portions (42, 43) is formed in such a manner as to extend in a direction intersecting a direction in which the slit (41) extends and is provided with a notch (44) which allows the projection (36) to enter and be engaged with the notch (44), and
wherein the locking portion (42, 43) includes the notch (44, 47) and an elastic engagement piece (46, 49) forming a peripheral part of the notch (44, 47).

2. The edged tool according to claim 1, **characterized in that** the locking portion (42) having the notch (44) is formed only at the end portion close to the grip portion (20) in the slit (41).

3. The edged tool according to claim 1, **characterized in that** the locking portion (42, 43) having the notch (44, 47) is formed at the end portion close to the grip portion (20) and at the end portion spaced away from the grip portion (20) in the slit (41).

4. The edged tool according to any one of claims 1 to 3, **characterized in that** the supporting portion (30) is smaller in cross section than the grip portion (20), and the proximal end portion of the protective cover (40) is in contact with the end face of the grip portion (20) when the protective cover (40) is placed at the exposing position.

5. The edge tool according to claim 1, **characterized in that** the protective cover (40) is placed so as to be rotatable aground the axis thereof, and that a plurality of manipulating projections (50) are projected at equal intervals on an outer peripheral surface of a proximal end portion of the protective cover (40) so as to rotate the protective cover (40) around an axis thereof for locking and unlocking the protective cover (40) at each of the covering position and the exposing position.

6. The edged tool according to any one of claims 1 to 5, wherein the protective cover (40) is placed so as to be rotatable around the axis thereof.

## Patentansprüche

1. Ein scharfes Werkzeug mit einer Schutzabdeckung (40), aufweisend einen Griffhauptkörper (10), der an einem distalen Ende einen Klingenkörper (35) aufweist, wobei die Schutzabdeckung (40) an einem Außenumfang des Griffhauptkörpers (10) angeordnet ist, um sich zwischen einer Position, in der die Schutzabdeckung (40) den Klingenkörper (35) bedeckt, und einer Position, in der der Klingenkörper (35) freigelegt ist, entlang einer Axialrichtung des Griffhauptkörpers (10) frei zu verschieben,
wobei der Griffhauptkörper (10) aufweist: einen Greifabschnitt (20), der ausgebildet ist, um von einer Hand gehalten zu werden, und einen Stützabschnitt (30), der einstückig mit dem Greifabschnitt (20) verbunden ist, um die Schutzabdeckung (40) abzustützen, um zu ermöglichen, dass sich die Schutzabdeckung (40) an dem Außenumfang verschiebt,
wobei ein Vorsprung (36) an dem Außenumfang des Stützabschnitts (30) ausgebildet ist,
wobei ein Paar von Verriegelungsabschnitten (42, 43) an der Schutzabdeckung (40) ausgebildet ist, wobei der Vorsprung (36) einen elastischen Eingriff mit den Verriegelungsabschnitten (42, 43) bildet, wenn die Schutzabdeckung (40) wahlweise in der Abdeckposition und in der Freilegposition angeordnet ist, wobei das scharfe Werkzeug **dadurch gekennzeichnet ist, dass**
ein Schlitz (41), der sich entlang der Axialrichtung der Schutzabdeckung (40) erstreckt, in der Schutzabdeckung (40) ausgebildet ist, der Vorsprung (36) in den Schlitz (41) gelangt und mit diesem in Eingriff ist, um bezüglich des Schlitzes (41) bewegbar zu sein,
wobei das Paar von Verriegelungsabschnitten (42, 43) an beiden Enden des Schlitzes (41) ausgebildet ist,
wobei mindestens einer der Verriegelungsabschnitte (42, 43) ausgebildet ist, um sich in einer Richtung zu erstrecken, die sich mit einer Richtung überkreuzt, in der sich der Schlitz (41) erstreckt, und mit einer Nut (44) versehen ist, die ermöglicht, dass der Vorsprung (36) in die Nut (44) gelangt und mit dieser in Eingriff ist, und
wobei der Verriegelungsabschnitt (42, 43) die Nut (44, 47) und ein elastisches Eingriffsstück (46, 49) aufweist, das einen Umfangsteil der Nut (44, 47) bildet.

2. Das scharfe Werkzeug gemäß Anspruch 1, **dadurch gekennzeichnet, dass** der Verriegelungsabschnitt (42), der die Nut (44) aufweist, nur an dem Endabschnitt nahe bei dem Greifabschnitt (20) in dem Schlitz (41) ausgebildet ist.

3. Das scharfe Werkzeug gemäß Anspruch 1, **dadurch gekennzeichnet, dass** der Verriegelungsabschnitt (42, 43), der die Nut (44, 47) aufweist, an dem Endabschnitt nahe bei dem Greifabschnitt (20) und an dem Endabschnitt im Abstand von dem Greifabschnitt (20) in dem Schlitz (41) ausgebildet ist.

4. Das scharfe Werkzeug gemäß irgendeinem der Ansprüche 1 bis 3, **dadurch gekennzeichnet, dass** der Stützabschnitt (30) im Querschnitt kleiner als der Greifabschnitt (20) ist und der proximale Endabschnitt der Schutzabdeckung (40) mit der Endfläche des Greifabschnitts (20) in Kontakt ist, wenn die Schutzabdeckung (40) in der Freilegposition angeordnet ist.

5. Das scharfe Werkzeug gemäß Anspruch 1, **dadurch gekennzeichnet, dass** die Schutzabdeckung (40) angeordnet ist, um um die Achse davon drehbar zu sein, und dass eine Mehrzahl von Betätigungsvorsprüngen (50) in gleichen Abständen an einer Außenumfangsfläche eines proximalen Endabschnitts der Schutzabdeckung (40) hervorsteht, um die Schutzabdeckung (40) um eine Achse davon zu drehen, um die Schutzabdeckung (40) in jeder von der Abdeckposition und der Freilegposition zu verriegeln und zu entriegeln.

6. Das scharfe Werkzeug gemäß irgendeinem der Ansprüche 1 bis 5, wobei die Schutzabdeckung (40) angeordnet ist, um um die Achse davon drehbar zu sein.

## Revendications

1. Outil de coupe avec un étui de protection (40) comprenant un corps principal de poignée (10) qui présente un corps de lame (35) au niveau d'une extrémité distale, dans lequel l'étui de protection (40) est placé sur une périphérie extérieure du corps principal de poignée (10), pour glisser librement le long d'une direction axiale du corps principal de poignée (10) entre une position où l'étui de protection (40) couvre le corps de lame (35) et une position où le corps de lame (35) est exposé ;
dans lequel le corps principal de poignée (10) comprend une partie de préhension (20) conçue pour être tenue d'une main et une partie de support (30) couplée d'une pièce à la partie de préhension (20) pour supporter l'étui de protection (40), afin de permettre à l'étui de protection (40) de glisser sur la périphérie extérieure ;
dans lequel une saillie (36) est formée sur la périphérie extérieure de la partie de support (30) ;
dans lequel une paire de parties de verrouillage (42, 43) sont formées sur l'étui de protection (40), dans lequel la saillie (36) réalise une mise en prise élastique avec les parties de verrouillage (42, 43) lorsque l'étui de protection (40) est placé de manière sélective au niveau de la position de couverture et au niveau de la position d'exposition, l'outil de coupe étant **caractérisé en ce que** :
une fente (41) qui s'étend le long de la direction axiale de l'étui de protection (40) est formée dans l'étui de protection (40), la saillie (36) pénètre dans la fente (41) et vient en prise avec celle-ci, de façon à pouvoir se déplacer par rapport à la fente (41) ;
dans lequel la paire de parties de verrouillage (42, 43) sont formées sur les deux extrémités de la fente (41) ;
dans lequel l'une au moins des parties de verrouillage (42, 43) est formée pour s'étendre dans une direction qui croise une direction dans laquelle la fente (41) s'étend, et est dotée d'une encoche (44) qui permet à la saillie (36) de pénétrer dans l'encoche (44) et de venir en prise avec celle-ci ; et
dans lequel la partie de verrouillage (42, 43) comprend l'encoche (44, 47) et un élément de mise en prise élastique (46, 49) qui forme une partie périphérique de l'encoche (44, 47).

2. Outil de coupe selon la revendication 1, **caractérisé en ce que** la partie de verrouillage (42) présentant l'encoche (44) est formée seulement au niveau de la partie d'extrémité à proximité de la partie de préhension (20) dans la fente (41).

3. Outil de coupe selon la revendication 1, **caractérisé en ce que** la partie de verrouillage (42, 43) présentant l'encoche (44, 47) est formée au niveau de la partie d'extrémité à proximité de la partie de préhension (20) et au niveau de la partie d'extrémité espacée de la partie de préhension (20) dans la fente (41).

4. Outil de coupe selon l'une quelconque des revendications 1 à 3, **caractérisé en ce que** la partie de support (30) présente une section transversale plus petite que celle de la partie de préhension (20), et la partie d'extrémité proximale de l'étui de protection (40) est en contact avec la face d'extrémité de la partie de préhension (20) lorsque l'étui de protection (40) est placé au niveau de la position d'exposition.

5. Outil de coupe selon la revendication 1, **caractérisé en ce que** l'étui de protection (40) est placé de façon à pouvoir tourner autour de l'axe de celui-ci, et **en ce qu'**une pluralité de saillies de manipulation (50) font saillie à intervalles égaux sur une surface périphérique extérieure d'une partie d'extrémité proximale de l'étui de protection (40) afin de faire tourner l'étui de protection (40) autour d'un axe de celui-ci, pour verrouiller et déverrouiller l'étui de protection (40) au niveau de la position de couverture et de la position d'exposition.

6. Outil de coupe selon l'une quelconque des revendications 1 à 5, dans lequel l'étui de protection (40) est placé de façon à pouvoir tourner autour de l'axe de celui-ci.
